# EUROPEAN PATENT APPLICATION

(11) **EP 4 437 982 A1**
(43) Date of publication of application: **02.10.2024**
(21) Application number: 23166067.1
(22) Date of filing: 31.03.2023
(51) Int. Cl.: A61B 17/34

(54) **A TROCAR FIXATION ASSEMBLY**

(71) Applicant: Mölnlycke Health Care AB, 402 52 Göteborg (SE)
(72) Inventor: ROSENGREN, Oscar, 436 55 HOVÅS (SE); GUNGNER, Mattias, 441 60 ALINGSÅS (SE)
(74) Representative: Kransell & Wennborg KB

(57) **Abstract**

The present disclosure generally relates to a trocar fixation assembly (100) comprising a dressing (101) and a trocar attachment device (102), wherein the dressing (101) has a first, skin-facing side (101a) and a second, opposing side (101b); the first, skin-facing side (101a) of the dressing (100) comprising an adhesive layer (103), wherein the dressing (101) comprises an aperture (104) for insertion of a trocar during use, characterized in that the dressing (101) further comprises a first annular coupling member (105) arranged on the second side (101b) of the dressing (101) and encircling the aperture (104); the first annular coupling member (105) being configured to detachably connect the dressing (101) with the trocar attachment device (102).

The present disclosure also relates to a trocar system (200) comprising the trocar fixation assembly (100).

## Description

### TECHNICAL FIELD

The present disclosure generally relates to a trocar fixation assembly comprising a dressing and a trocar attachment device. The present disclosure also relates to a trocar system comprising the trocar fixation assembly.

### BACKGROUND

Minimal invasive surgery (MIS) is a technique for performing surgery that is associated with less pain, shorter recovery and fewer complications compared to traditional open surgery.

In an MIS procedure, a small incision is typically made through the skin of a patient, and a trocar is thereafter inserted into the incision. The trocar serves as a working channel that allows for the entry of various surgical instruments and cameras into a body cavity, e.g. an abdominal cavity.

A trocar comprises a cannula, which is a hollow and tube-shaped shaft that is placed inside the patient to provide access to the body cavity during the MIS procedure. An obturator is typically used to allow the cannula to penetrate the body cavity for initial placement. When the cannula has entered the body cavity, the obturator is removed.

During some MIS procedures, e.g. laparoscopic procedures, carbon dioxide is utilized to fill the body cavity such that the organs and other structures may be more easily observed and accessed.

During an MIS procedure, a plurality of surgical instruments and tools are typically utilized. One challenge during MIS procedures is that the trocar may be dislocated during surgery; i.e. that the trocar may slip out from the body cavity or that the trocar projects too deep into the body cavity. This may result in surgical failure and is also associated with risks for the patients.

To date, the commonly used trocars in MIS procedures include balloon trocars, and Hasson trocars, respectively. A Hasson trocar is a non-bladed trocar that stays in place by means of sutures anchored to the abdominal wall fascia. A balloon trocar comprises a balloon at the end of the trocar that is inflated with air. Instead of utilizing sutures for fixing the trocar, the inflated balloon anchors the trocar inside the abdomen.

Fixating and stabilizing the trocar, particularly the cannula, is typically challenging during most MIS procedures. Both the balloon and Hasson trocar system suffer from disadvantages with respect to fixation and stabilization. For example, the inflated balloon of a balloon trocar may rupture, which is associated with risks for the patient. Furthermore, a balloon trocar cannot simply be removed from the body cavity since the balloon must first be deflated.

The Hasson trocar is cumbersome in the sense that fixation and stabilization require the aid of sutures to fixate the trocar in the body cavity. Upon removal of the trocar, the sutures must first be removed. Furthermore, the cannula must typically comprise ridges to fix the skin mechanically.

Another challenge during MIS procedures is sterility and keeping the surgical site and the area circumventing the trocar clean and free from blood. It is also important to prevent carbon dioxide from leaking out.

Accordingly, there is room for improvements in fixating and stabilizing the trocar in a sterile, simplified and less cumbersome manner. Such fixation and stabilization means should prevent dislocation of the trocar cannula during use and facilitate initial attachment, as well as detachment of the trocar from the patient's skin.

### SUMMARY

In view of the above-mentioned problems, it is an object of the present disclosure to provide improvements with respect to relieving the burden for surgical staff and the patients and providing a facilitated and improved means to handle and fixate a trocar during minimal invasive surgery.

According to a first aspect, there is provided a trocar fixation assembly comprising a dressing and a trocar attachment device, wherein the dressing has a first, skin-facing side and a second, opposing side; the first, skin-facing side of the dressing comprising an adhesive layer, wherein the dressing comprises an aperture for insertion of a trocar during use, wherein the dressing further comprises a first annular coupling member arranged on the second side of the dressing and encircling the aperture; the first annular coupling member being configured to detachably connect the dressing with the trocar attachment device.

The present disclosure is based on the realization that a trocar fixation assembly, as defined hereinabove, greatly facilitates the minimally invasive surgical (MIS) procedure for the surgeon and for the surgical staff. A simplified means for stabilizing the trocar is provided, and contamination as well as undesired trocar dislocation during use are prevented.

The dressing may initially be attached to the skin of a patient in an area where the MIS procedure is to be accomplished. The dressing comprises an adhesive layer on its skin-facing side, which secures a tight fit to the skin. The dressing is fixed in place in the area circumventing the surgical site. The dressing comprises an aperture, which is typically centrally disposed in the dressing. When the dressing has been attached, the surgeon may puncture the skin, through the aperture of the dressing, and subsequently connect a trocar attachment device to the dressing by means of the first annular coupling member. The dressing surrounds the incision site and forms a seal against blood exuded from the wound site. After connection of the trocar attachment device to the dressing, a trocar may be inserted into and attached to the trocar attachment device, through the aperture of the dressing and through the punctured skin site. Alternatively, the trocar is already attached to the trocar attachment device prior to connection with the dressing.

The trocar fixation assembly of the present disclosure allows for an easy and quick assembly and disassembly of the components. For example, upon completion of a specific procedure, the trocar and the trocar detachment device may easily be removed, leaving the dressing on the skin of the patient. The dressing secures a sterile environment in the area surrounding the incision site. The dressing may subsequently be removed, in a separate and more gentle manner from the skin of patient. Alternatively, the dressing may remain in place in case the surgeon needs to re-attach the trocar attachment device and reinsert the trocar.

Since no part of the trocar fixation assembly remains inside the body (as is the case with balloon trocars) or requires fixation by means of sutures to the skin (as is the case with Hasson trocars), the trocar fixation assembly provides a safe and simplified means to fixate a trocar in position during minimal invasive surgery. Furthermore, the attachment and detachment of the trocar fixation assembly is significantly facilitated.

In exemplary embodiments, the trocar attachment device comprises a second annular coupling member configured to engage with the first annular coupling member of the dressing to provide a detachable connection between the dressing and the trocar attachment device.

The annular first and second coupling members form a tight seal between the dressing and the trocar attachment device, yet allow for an easy and quick detachment and decoupling from one another.

The detachable connection between the first and second annular coupling members is not limited to a specific type of coupling mechanism, but any mechanism that allows for the first and second annular coupling members to be connected is conceivable.

For example, the second annular coupling member may be configured to engage with the first annular coupling member by means of a snap-fit mechanism, a bayonet coupling or by a screwing mechanism.

These coupling mechanisms allow for a simple and quick attachment and detachment of the first and second annular coupling members.

In exemplary embodiments, the first annular coupling member comprises an axially extending wall portion and an annular collar portion extending radially outwards from the wall portion.

The annular collar portion of the first annular coupling member allows for a snap-fit connection between the first and second annular coupling members. When the second annular coupling member has been attached to the first annular coupling member, the annular collar portion secures that the second annular coupling members it is kept in place and is prevented from detachment from the first annular coupling member.

In exemplary embodiments, the second annular coupling member comprises a first collar portion and a second collar portion and a connecting wall portion defined between the first and the second collar portions, wherein the radial circumference of each of the first and the second collar portion is larger than the radial circumference of the wall portion, wherein the second annular coupling member further comprises a locking ring arranged between the first and the second collar portions; the locking ring being configured to be shifted between a locked position and an unlocked position, wherein in the locked position, the radial circumference of the locking ring is smaller than the radial circumference of the locking ring in the unlocked position.

The locking ring secures that the trocar attachment device remains firmly attached to the dressing during an MIS procedure. In the locked position, the locking ring prevents the first and second coupling members from being released from each other. A tight fit between the dressing and the trocar attachment device is thus provided, yielding a safe and reliable trocar attachment assembly.

When the surgery is completed, the locking ring may easily be shifted to the unlocked position, and the first and second locking members may be detached such that the trocar attachment device can be removed from the dressing.

In exemplary embodiments, the second annular coupling member of the trocar attachment device comprises a sealing member extending from the second annular coupling member to an upper portion, wherein the upper portion is configured to be attached to a trocar during use.

The sealing member forms a seal between the skin site and the trocar and prevents fluid and gas leakage into and out of the interior cavity of the patient. It also prevents external substances and contaminants from entering the incision site and the skin-site surrounding the incision site.

The upper portion is configured to be attached to the cannula of the trocar.

Preferably, the sealing member comprises an elastomeric material.

Accordingly, the sealing member is flexible and deformable. This is beneficial since it allows the trocar to be tilted and arranged in various angled positions. In many situations, the surgeon needs to reach and operate in a multitude of positions within the cavity, both near the skin surface and deeper down in the body cavity. The flexible and elastomeric sealing member allows for the trocar (and thus also any instrument inserted into the trocar) to be tilted, angled and inserted deeper into the body cavity, which greatly supports the surgeon during the MIS procedure. It also restricts the trocar from penetrating too deep into the body cavity. Furthermore, it prevents dislocation of the trocar during use.

Furthermore, the trocar fixation assembly allows for a trocar cannula to be fixed in a position very close to the skin surface, which is beneficial during e.g. hernia repair surgery.

In exemplary embodiments, the first annular coupling member may have a perimeter, pₐₙₙ, being from 100% to 500%, preferably from 200% to 400% larger than the maximum perimeter, p_{troc}, of a trocar cannula inserted into the trocar fixation assembly.

Hence the sealing member tapers from the second annular coupling member towards the upper portion attached to the trocar, i.e. the trocar cannula.

This configuration is beneficial as it leaves sufficient room and space inside the sealing member such that a variety of angled manipulations may be accomplished with the trocar during use.

In exemplary embodiments, the sealing member is symmetric about a longitudinal center line and has a maximum longitudinal extension of from 15 to 100 mm, preferably from 20 to 60 mm.

If the longitudinal extension; i.e. height, is too small, then the angular manipulations of the trocar become more limited. In contrast, if the longitudinal extension is too high, then this may cause the trocar to penetrate too deep into the body cavity, and consequently harm any organs therein.

In exemplary embodiments, the sealing member is transparent.

This allows for the surgeon to observe and view the incision site and the skin site surrounding the incision site. For example, any bleeding or leakage from the incision site may be monitored. Accordingly, a more controlled MIS procedure is provided.

In exemplary embodiments, the upper portion of the trocar attachment device is configured to be detachably attached to the trocar during use.

This is advantageous since the trocar may be attached to and detached from the trocar attachment device in a simple manner. For example, in certain situations, it may be beneficial to leave the dressing and the trocar attachment device on the skin of a patient, remove the trocar and continue the operation at a later point in time. In many MIS procedures, a plurality of trocars is used, which may then be used in conjunction with a plurality of trocar fixation assemblies. The surgeon typically alternates between these assemblies and trocars and may choose which ones to operate from at different points in time.

The detachable configuration between the trocar attachment device and the trocar also secures a more gentle and less harmful removal of the trocar system from the patient. First, the trocar may gently be removed, subsequently, the trocar attachment device may be removed by simply disconnecting the second annular coupling member from the first annular coupling member, and finally, the dressing may be removed from the skin of the patient.

In exemplary embodiments, the upper portion may comprise an annular element defining a channel for receiving a trocar; the annular element being disposed about a longitudinal center line; the annular element having a first, open configuration, which allows for a trocar to be moved along the longitudinal center line of the annular element, and a second, fixed configuration, which allows for a trocar to be fixed in a locked position.

This configuration allows for the trocar to be fixed in position at a specific depth inside the body cavity. The penetration depth of the trocar cannula to be inserted into the body cavity may thus be adjusted and locked in a desirable position. For example, the trocar may be fixed at a position close to the skin surface, but still be angled by means of the elastic sealing member. This may be beneficial during e.g. hernia surgery. Furthermore, the trocar is prevented from penetrating too deep into the body cavity. The depth of trocar insertion into the body cavity may easily be adjusted and varied during the surgical procedure.

In exemplary embodiments, the second side of the dressing comprises a backing layer, wherein the dressing further comprises an absorbent pad between the backing layer and the adhesive layer.

The adhesive layer secures a tight fit to the skin, and the dressing is fixed in place in the area circumventing the surgical site. Blood and body fluids exuded from the surgical site may be evaporated through the backing layer.

An absorbent pad is beneficial to absorb the blood resulting from the surgical intervention. It is desirable to prevent blood from accumulating at the skin, and instead secure removal and proper handling of the blood by means of the absorbent pad. Accordingly, contaminating microorganisms are prevented from accumulating at the skin, and the sterility is improved.

In exemplary embodiments, the aperture of the dressing is covered by an incision film configured to be cut prior to or during insertion of a trocar into the aperture.

An incision film is a film which is designed to be cut through during surgery. The incision film prevents the incision site from bacterial contamination and seals the area around the trocar cannula. Prior to surgery, the body cavity, e.g. the abdomen is typically insufflated with a gas, typically carbon dioxide, to produce a pneumoperitoneum. Creating a pneumoperitoneum allows for separation between the body wall and internal organs and thereby allows for an internal working space for manipulation of organs by surgical instruments.

It is important to prevent gas from leaking out of the body. The provision of an incision film may prevent undesired leakage of gas from the incision site.

In exemplary embodiments, the incision film comprises markings indicating where and/or how to cut the incision film.

When inserting a trocar into the skin of a patient, it is important that the length of the incision matches that of the trocar diameter. Otherwise, there is an enhanced risk for the trocar to slip out of the body cavity. Furthermore, there is an enhanced risk for contamination. There is also a risk for leakage of gas, e.g. carbon dioxide from the body cavity.

A common practice during MIS procedures is that the surgical staff, prior to surgery, presses the trocar onto the skin at the desired insertion point to create a temporary marking on the skin. The surgeon can thereafter use that marking to determine the boundaries for the incision. By providing markings onto the incision film, a direct guidance is given as to where and/or how to cut the incision film to secure a correct size of the incision. The markings may match the diameter of commonly used trocars. Accordingly, the risk of making a too large or too small incision prior to trocar insertion is minimized.

In exemplary embodiments, the dressing comprises a gripping tab extending radially from an outer periphery of the dressing.

The gripping tab facilitates the removal of the dressing from the skin of a patient.

According to another aspect, there is provided a trocar system comprising the trocar fixation assembly according to any one of the preceding claims and a trocar.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a trocar and a trocar fixation assembly according to an exemplary embodiment of the present disclosure, applied to the skin of a patient.
Figure 2A illustrates trocar fixation assembly according to an exemplary embodiment of the present disclosure in conjunction with a trocar
Figure 2B illustrates a split view of the trocar fixation assembly in figure 2A. including a zoomed-in, cross-sectional view of the dressing.
Figure 2C illustrates a cross-sectional view of the trocar fixation assembly in figure 2A.
Figure 3 illustrates a dressing comprising an aperture covered by an incision film according to an exemplary embodiment of the present disclosure.
Figures 4A-D illustrate how a trocar can be operated in various angular positions by means of a trocar attachment assembly according to an exemplary embodiment of the present disclosure.
Figure 5 illustrates an alternative embodiment of a trocar fixation assembly of the present disclosure applied to the skin of a patient.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

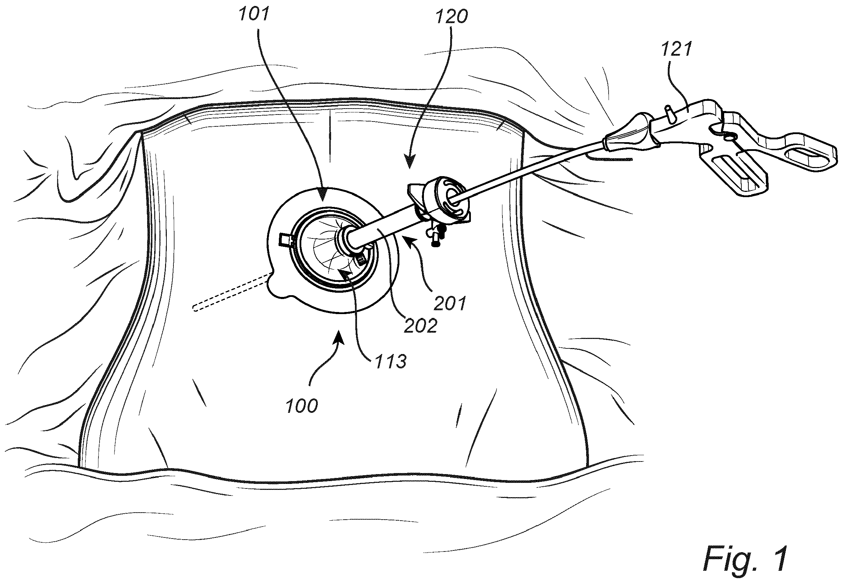

With reference to figure 1, a trocar fixation assembly according to an exemplary embodiment of the present disclosure is schematically illustrated.

The trocar fixation assembly 100 has been attached to the skin 120 of a patient. The trocar attachment assembly 100 is attached to a trocar 201 comprising a trocar cannula 202 through which a surgical instrument 121 is inserted.

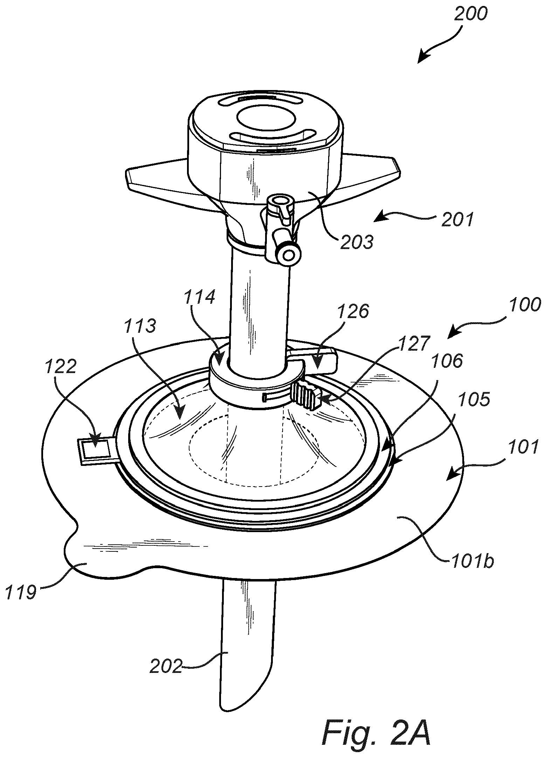

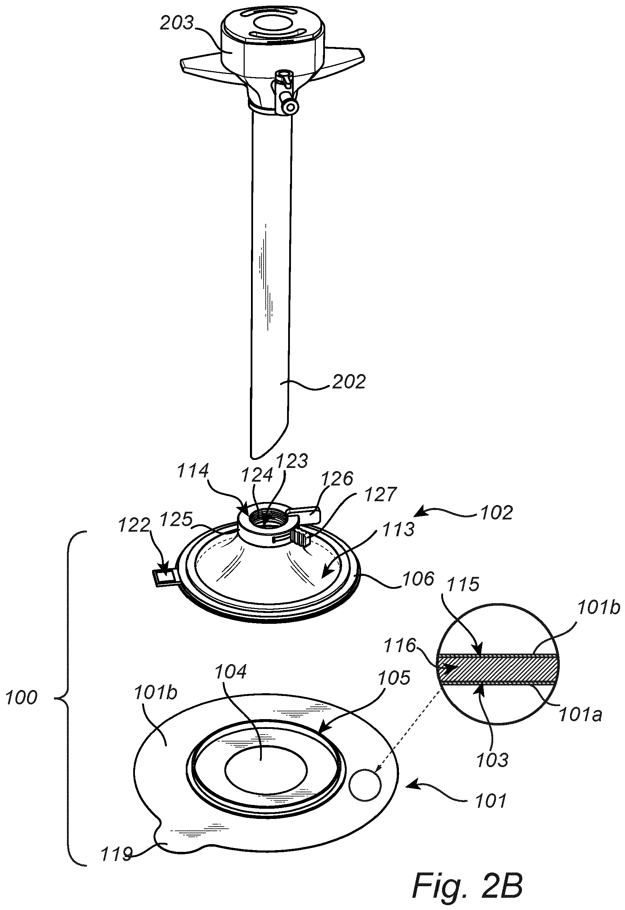

As best illustrated in figures 2A and 2B, the trocar fixation assembly 100 comprises a dressing 101 and a trocar attachment device 102, wherein the dressing 101 has a first, skin-facing side 101a and a second, opposing side 101b; the first, skin-facing side 101a of the dressing 100 comprising an adhesive layer 103, wherein the dressing comprising an aperture 104 for insertion of a trocar during use, and wherein the dressing 101 further comprises a first annular coupling member 105 arranged on the second side 101b of the dressing 101 and encircling the aperture 104; the first annular coupling member 105 being configured to detachably connect the dressing 101 with the trocar attachment device 102.

As used herein, the term "trocar fixation assembly" means an assembly comprising at least a trocar attachment device and a dressing. The dressing is detachably attached to the trocar attachment device. The trocar fixation assembly is to be used in conjunction with a trocar. More particularly, the trocar fixation assembly is used to receive and engage with a trocar cannula.

As used herein, the term "trocar" means a device through which a minimal invasive surgery can be accomplished. The trocar typically comprises at least a cannula, i.e. a hollow tube shaped member, and a gas-tight valve (see 203 in figure 2B). The trocar allows for the entry of surgical instruments, such as graspers, scissors, staplers, cameras etc.

The first annular coupling member 105 is configured to detachably connect the dressing 101 with the trocar attachment device 102.

Hence, in use, the trocar attachment device 102 is preferably firmly attached to the dressing, but when the surgery is completed, the components may easily be detached from one another, yielding a safe and gentle mode of removal of the trocar fixation assembly. The detachable connection between the dressing 101 and the trocar attachment device 102 allows for an easy, and quick assembly and disassembly of the components. It also allows for a safe and stable means for fixating a trocar during use.

As illustrated in e.g. figure 2B, the dressing 101 comprises an aperture 104 through which a trocar is to be inserted. The aperture 104 is preferably arranged in a central portion of the dressing 101. The aperture may be a centrally disposed aperture. The size of the aperture 104 of the dressing is larger than the size of the trocar cannula 202.

The aperture 104 may be circular. The diameter of the aperture is thus larger than the maximum diameter of a trocar cannula. For example, the diameter of the aperture may be in the range of from 10 to 70 mm, e.g. from 15 to 50 mm.

In the figures, the dressing 101 is illustrated as circular, however the dressing is by no means limited to a specific shape. Any shape is conceivable.

The first annular coupling member 105 is annular; i.e. ring-shaped and encircles the aperture of the dressing. The first annular coupling member 105 typically extends uninterrupted around the aperture 104 of the dressing 101.

The first annular coupling member 105 is not limited to a specific material but is preferably rigid enough to enable firm attachment with a trocar attachment device. For example, the first annular coupling member 105 may comprise a plastic material.

The first annular coupling member 105 may e.g. be attached to the second side 101b of the dressing 101 by adhesion or welding.

The first annular coupling member 105 may have a diameter in the range of from 30 to 70 mm, e.g. from 40 to 60 mm. The perimeter of the first annular coupling member 105 may be from 90 to 220 mm, e.g. from 125 to 190 mm. The height of the annular member may be in the range of from 5 to 25 mm, e.g. from 8 to 15 mm.

As illustrated in e.g. figure 2A, the dressing 101 may form a closed path around the trocar attachment device 102. Accordingly, the dressing 101 may extend uninterrupted around the trocar attachment device 102. Hence, no part of the dressing is openable after the dressing has been applied to the skin. This is beneficial to secure a tight seal to the skin and also to prevent contaminants from entering the incision or the area of the skin circumventing the incision.

The trocar attachment device 102 may comprise a second annular coupling member 106 configured to engage with the first annular coupling member 105 of the dressing 100 to provide a detachable connection between the dressing 101 and the trocar attachment device 102.

The second annular coupling member 106 is annular; i.e. ring-shaped. The second annular coupling member 106 is not limited to a specific material. For example, a plastic material may be utilized.

The second annular coupling member 106 may be configured to engage with the first annular coupling member 105 by means of a snap-fit mechanism, a bayonet coupling or by a screwing mechanism.

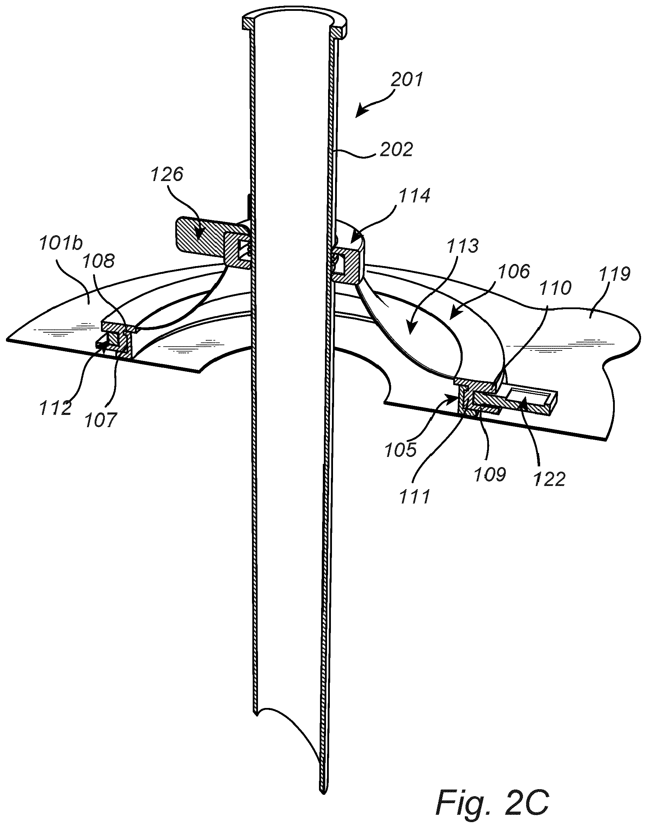

As best illustrated in figure 2C, the first annular coupling member 105 may comprise an axially extending wall portion 107 and an annular collar portion 108 extending radially outwards from the wall portion 107.

As used herein, an axial direction is the direction of the trocar fixation assembly when applied to the patient. The axial direction is perpendicular to the skin surface of the user.

The radial direction is transverse to the axial direction.

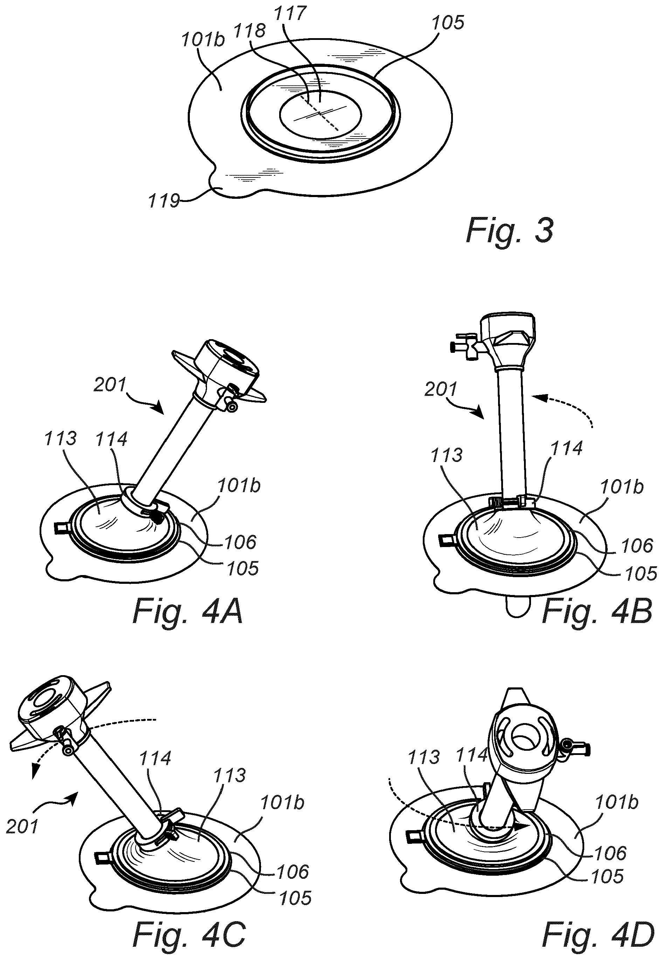

The annular collar portion 108 has a radial circumference that is larger than the radial circumference of the wall portion 107. Hence, the annular collar portion 108 forms a flange portion that may lock the second coupling member 106 of the trocar attachment device 102 during the MIS procedure. The second coupling member 106 may thus be attached to the first coupling member 105 by means of a snap-fit mechanism. In figure 3, the annular collar portion 108 is attached to the second coupling member 106 by means of an indentation formed in the second coupling member 106. However, it is equally conceivable that the second coupling member is kept in place by arranging the second coupling portion 106 below the annular collar portion 108 of the first coupling member 105.

The second annular coupling member 106 may comprise a first collar portion 109 and a second collar portion 110 and a connecting wall portion 111 defined between the first 109 and the second 110 collar portions, wherein the radial circumference of the first 109 and the second 110 collar portion is larger than the radial circumference of the wall portion 111, wherein the second annular coupling member 106 further comprises a locking ring 112 arranged between the first 109 and the second 110 collar portions; the locking ring 112 being configured to be shifted between a locked position and an unlocked position, wherein in the locked position, the radial circumference of the locking ring 112 is smaller than the radial circumference of the locking ring 112 in the unlocked position.

The locking ring 112 may comprise a tab 122 which may shift the locking ring 112 between a locked and an unlocked position. The tab may comprise a first and a second tab portion. A first distal end of the locking ring may be connected to the first tab portion and a second distal end of the locking ring may be connected to the second tab portion.

In figure 2A, the locking ring is in the locked position, and the first and second tab portions are connected. When the tab is pushed, the first and the second tab portions are disconnected and may be moved apart which causes the radial circumference of the locking ring 112 to be enlarged and the locking ring to adopt an unlocked position. The pushing of the tab may be associated with a click, which indicates to the user that the locking ring has been locked (or unlocked) correctly. The locking/unlocking may be performed by a one-hand-operation.

As illustrated in e.g. figure 2A, the second annular coupling member 106 of the trocar attachment device 102 may comprise a sealing member 113 extending from the second annular coupling member 106 to an upper portion 114, wherein the upper portion 114 is configured to be attached to a trocar 201 during use.

The sealing member 113 provides a seal between the skin site and the trocar. It also prevents fluid and gas leakage from the incision site to the surrounding.

The sealing member 113 preferably comprises an elastomeric material.

An "elastomeric material" is any material exhibiting elastic or rubber-like properties. The elastomeric material may be a natural or synthetic polymer having elastic properties. Examples of elastomeric materials include silicone, natural rubber or synthetic rubber or a combination thereof.

The elastomeric material of the sealing member 113 has a high elongation rate and is resilient such that it recovers from an elastic deformation. In other words, after the sealing member 113 has been subjected to a deformation force, it returns to its original conformation.

The sealing member is flexible and deformable. As illustrated in figures 4a-4d, the trocar may be tilted and angled in any direction during operation which significantly facilitates the MIS procedure for the surgeon.

The first annular coupling member 105 may have a perimeter, pₐₙₙ being from 100% to 500%, preferably from 200% to 400% larger than the maximum perimeter, p_{troc} of a trocar cannula inserted into the trocar fixation assembly.

The perimeter of a trocar cannula typically ranges from 4 to 20 mm, e.g. from 5 to 15 mm.

As illustrated in e.g. figure 2B, the sealing member 113 tapers in width and in height from the second annular coupling member 106 towards the upper portion 114 attached to the trocar, i.e. the trocar cannula.

This is beneficial since it allows the surgeon to angle, tilt, and shift the position of the trocar during operation.

The sealing member may be symmetric about a longitudinal center line and typically has a maximum longitudinal extension of from 15 to 100 mm, e.g. from 25 to 50 mm.

The maximum longitudinal extension corresponds to the height of the sealing member.

The maximum lateral extension of the sealing member corresponds to the diameter of the second annular coupling member.

These dimensions are beneficial to secure a plurality of tilted or angled positions of the trocar during an MIS procedure.

The sealing member 113 may be substantially transparent.

This allows for the surgeon to observe and view the incision site and the skin site surrounding the incision site. This is beneficial to improve the precision and control during the MIS procedure.

The "upper portion" of the trocar attachment device is configured to be attached to the trocar 201 during use.

Preferably, the upper portion 114 is detachably attached to the trocar during use. This allows for a flexible attachment and detachment of all components of a trocar system incorporating a trocar fixation assembly of the present disclosure.

The present disclosure is not limited to a specific mechanism for detachable attachment between the upper portion 114 of the trocar attachment device and the trocar, but any mechanism or technique may be utilized.

For example, as illustrated in figure 2B, the upper portion 114 may comprise an annular element defining a channel 123 for receiving a trocar 201; the annular element being disposed about a longitudinal center line; the annular element having a first, open configuration, which allows for a trocar to be moved along the longitudinal center line of the annular element, and a second, fixed configuration, which allows for a trocar to be fixed in a locked position.

The annular element may comprise a spring member, e.g. a spring wire configured to move the fixation device between the first and the second configurations.

The annular element may comprise interior walls 124 and exterior walls 125, wherein the spring member; i.e. spring wire is arranged to encircle at least a portion of the interior walls 124 of the annular element.

In the area where the spring wire is arranged, the interior walls 124 of the annular element may comprise one or more annular indentations. The annular indentations allow for the spring wire to retract such that the cross-sectional area of the channel 123 is increased, and such that the first, open configuration can be adopted.

In the second, fixed configuration, the cross-sectional area of at least one portion of the channel 123 (i.e. where the spring wire is arranged) is smaller than the cross-sectional area of same portion of the channel 123 in the first, open configuration. The radially projecting portion of the spring wire prevents a trocar from being inserted into the channel 123. It also secures a clamping mechanism towards a trocar cannula inserted into the channel 123.

The spring member; i.e. spring wire may be operably engaged with at least a first locking tab 126. The first locking tab 126 may project radially from the exterior walls 125 of the annular element. For example, a first distal end of the spring wire may be connected to or form part of the first locking tab 126. A second distal end of the spring wire may be utilized to anchor the spring wire at the interior walls 124 of the annular element.

The annular element may comprise a first 126 and a second locking tab 127. As illustrated in figure 2B, the second locking tab 127 projects radially from the exterior walls 125 of the annular element.

The first locking tab 126 may be movable in a circumferential direction of the annular element. The second locking tab 127 may be movable or it may be fixed. The second locking tab 127 may serve as a support to facilitate the movement of the first locking tab 126 between the first and the second positions in order to facilitate shifting between the first, and the second configurations of the annular element.

The first and the second tabs enable the annular element to operate between a first, open configuration, and a second, fixed configuration.

As mentioned hereinabove, the first, skin-facing side 101a of the dressing 100 comprises an adhesive layer 103.

The adhesive layer 103 may comprise any skin-compatible adhesive.

Preferably, the adhesive layer 103 comprises a silicone-based adhesive.

A silicone-based adhesive is gentle to the skin and may be removed and applied to the skin in a gentle manner, without causing any trauma. For example, the adhesive layer 103 may comprise a silicone gel. The silicone gel may be provided as a coating on the backing layer or a pad, if present.

The adhesive layer 103 may comprise one or more sub-layers. For example, the adhesive layer 103 may comprise a polymeric film and an adhesive silicone gel layer, wherein the adhesive silicone gel layer is arranged to contact the skin.

The adhesive layer may comprise a plurality of perforations. The perforations facilitate the entry of wound exudate into the dressing.

As illustrated in the zoomed-in view in figure 2B the second side 101b of the dressing 101 may comprise a backing layer 115 and wherein the dressing further comprises an absorbent pad 116 arranged between the backing layer 115 and the adhesive layer 103.

The absorbent pad 116 keeps the area circumventing the incision and the inserted trocar free from blood. The absorbent pad 116 absorbs the blood and prevents maceration at the surgical site. Furthermore, the absorbent pad prevents contaminating microorganisms from accumulating at the skin or incision site. In embodiments where the dressing comprises a pad, the backing layer 115 may be adhesively attached to the pad. Alternatively, the backing layer 115 may be laminated to the pad. For example, heat lamination may be utilized to apply the backing layer 115 to the absorbent pad 116.

The absorbent pad 116 preferably comprises a polyurethane foam. Typically, the polyurethane foam is a hydrophilic polyurethane foam. A polyurethane foam has a pressure relieving effect and is capable of absorbing large amounts of fluids.

The absorbent pad 116 may comprise one or more layers. If the pad comprises a plurality of pad-forming layers, the pad-forming layers may be laminated or attached to each other.

In embodiments where the dressing comprises an absorbent pad, the absorbent pad comprises a centrally disposed aperture, wherein the aperture of the absorbent pad is aligned with the aperture of the backing layer and the adhesive layer. The aperture of the dressing may extend through the backing layer, absorbent pad and the adhesive layer (as illustrated in the figures).

The surface area of the backing layer 115 typically corresponds to the surface area of the adhesive layer 103. Accordingly, the backing layer and the adhesive layer are coextensive.

The absorbent pad may have a surface area corresponding to the surface area of the backing layer and the adhesive layer.

Alternatively, the absorbent pad may have a smaller surface area than the surface area of the backing layer 115 and the adhesive layer 103. Accordingly, the backing layer and the adhesive layer 103 may extend beyond the contour of the absorbent pad to form a border portion. This construction may be beneficial to improve the adhesion to the skin and prevent the dressing from detaching from the skin.

In exemplary embodiments, and as illustrated in figure 4, the aperture 104 may be covered by an incision film 117 configured to be cut prior to or during insertion of a trocar into the aperture 104.

As used herein, the term "incision film" means a film used during surgery, and which is designed to be cut through. The incision film, which may also be referred to as a "surgical incision film" improves the sterility at the surgical site and minimizes leakage of carbon dioxide from the body cavity.

The incision film 117 may be attached to a portion of the adhesive layer. Alternatively, the incision film 117 may be arranged between the backing layer and the adhesive layer. The incision film 117 comprises a proximal side facing the skin of a patient, and an opposing distal side facing away from the skin of a patient. The proximal side of the incision film may be adhesive or non-adhesive.

### The incision film may e.g. comprise polyurethane, polyester and/or polypropylene

As illustrated in figure 4, the incision film 117 may comprise markings 118 indicating where and/or how to cut the incision film 117.

One challenge during MIS procedures is to puncture the skin of a patient correctly prior to insertion of the trocar. If a too large incision is made in the skin, carbon dioxide inflated into the body cavity prior to surgery may escape from the incision. If a too small incision is made, it may be difficult and harmful to the patient when the trocar is to be inserted.

The markings 118 indicate to the caregiver the correct length of the incision; i.e. a length that substantially matches the diameter of a trocar cannula. In figure 4, the markings 118 are provided in the form of a dotted cross. The size of the cross may match the size of a conventional trocar cannula.

The markings are not limited to a specific shape, but any markings that guides the caregiver to correctly puncture the skin may be utilized.

According to another aspect, there is provided a trocar system 200 comprising a trocar fixation assembly 100 as described hereinbefore and a trocar 201.

As illustrated in e.g. figure 2A, the trocar 201 comprises a cannula 202. The cannula serves as the "working channel" through which a variety of surgical tools can be inserted.

The cannula 202 is typically a hollow tube that serves as channel for the insertion of surgical tool. The cannula may be made of plastic or metal. The cannula may comprise a gas-tight valve 203 that provides for an internal air-seal allowing instruments to move in and out of the cannula without loss of pneumoperitoneum; i.e. gas present in the body cavity. The valve may be manually or automatically retractable during instrument passage.

The cannula 202 typically comprises an interior surface and an exterior surface. The exterior surface of the cannula 202 may comprise ridges or threads or may be smooth.

The tip of the cannula may be conical, blunt, eccentric or pyramidal to facilitate penetration and entry into a body cavity.

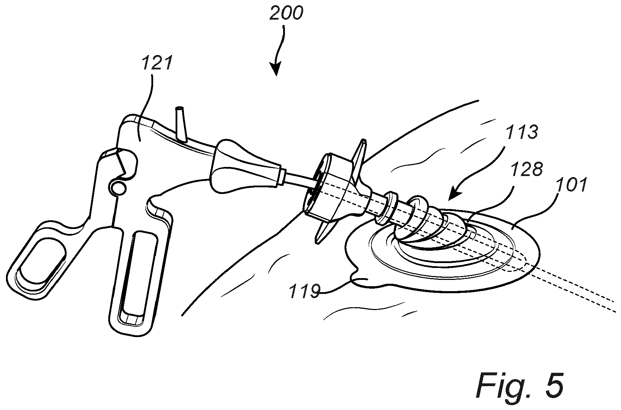

Figure 5 illustrates a trocar system 200 according to an exemplary embodiment of the present disclosure.

The trocar system 200 comprises a trocar and a trocar fixation assembly 100.

The trocar fixation assembly 100 is attached to the skin of a patient by means of the adhesive layer of the dressing 101. The sealing member 113 forms a seal against fluid and/or gas. The sealing member 113 comprises an elastomeric material to facilitate bending of the trocar 201.

In this embodiment, the sealing member 113 comprises a plurality of pleats 128, which enhance the ability of the sealing member 113 to bend and become angled in a variety of positions.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A trocar fixation assembly (100) comprising a dressing (101) and a trocar attachment device (102), wherein said dressing (101) has a first, skin-facing side (101a) and a second, opposing side (101b); said first, skin-facing side (101a) of said dressing (100) comprising an adhesive layer (103), wherein said dressing (101) comprises an aperture (104) for insertion of a trocar during use, **characterized in that** said dressing (101) further comprises a first annular coupling member (105) arranged on said second side (101b) of said dressing (101) and encircling said aperture (104); said first annular coupling member (105) being configured to detachably connect said dressing (101) with said trocar attachment device (102).

2. The trocar fixation assembly (100) according to claim 1, wherein said trocar attachment device (102) comprises a second annular coupling member (106) configured to engage with said first annular coupling member (105) of said dressing (100) to provide a detachable connection between said dressing (101) and said trocar attachment device (102).

3. The trocar fixation assembly (100) according to claim 2, wherein said second annular coupling member (106) is configured to engage with said first annular coupling member (105) by means of a snap-fit mechanism, a bayonet coupling or by a screwing mechanism.

4. The trocar fixation assembly (100) according to any one of the preceding claims, wherein said first annular coupling member (105) comprises an axially extending wall portion (107) and an annular collar portion (108) extending radially outwards from said wall portion (107).

5. The trocar fixation assembly according to any one of claims 2-4, wherein said second annular coupling member (106) comprises a first collar portion (109) and a second collar portion (110) and a connecting wall portion (111) defined between said first (109) and said second (110) collar portions, wherein the radial circumference of each of said first (109) and said second (110) collar portion is larger than the radial circumference of said wall portion (111), wherein said second annular coupling member (106) further comprises a locking ring (112) arranged between said first (109) and said second (110) collar portions; said locking ring (112) being configured to be shifted between a locked position and an unlocked position, wherein in said locked position, the radial circumference of said locking ring (112) is smaller than the radial circumference of said locking ring (112) in said unlocked position.

6. The trocar fixation assembly (100) according to any one of claims 2-5, wherein said second annular coupling member (106) of said trocar attachment device (102) comprises a sealing member (113) extending from said second annular coupling member (106) to an upper portion (114), wherein said upper portion (114) is configured to be attached to a trocar (201) during use.

7. The trocar fixation assembly (100) according to claim 6, wherein said sealing member (113) comprises an elastomeric material.

8. The trocar fixation assembly (100) according to any one of the preceding claims, wherein said first annular coupling member (105) has a maximum perimeter, pₐₙₙ, being from 100% to 500%, preferably from 200 to 400 % larger than the maximum perimeter, p_{troc}, of a trocar cannula (202) inserted into said trocar fixation assembly (100).

9. The trocar fixation assembly (100) according to any one of claims 6-8, wherein said sealing member (113) is symmetric about a longitudinal center line and has a maximum longitudinal extension of from 15 to 100 mm, e.g. from 20 to 60 mm.

10. The trocar fixation assembly (100) according to any one of claims 6-9, wherein said sealing member (113) is transparent.

11. The trocar fixation assembly (100) according to any one of claims 6-10, wherein said upper portion (114) of said trocar attachment device is configured to be detachably attached to a trocar (201) during use.

12. The trocar fixation assembly (100) according to any one of claims 6-11, wherein said upper portion (114) comprises an annular element defining a channel (123) for receiving a trocar (201); said annular element being disposed about a longitudinal center line, wherein said annular element has a first, open configuration, which allows for a trocar to be moved along said longitudinal center line of said annular element, and a second, fixed configuration, which allows for a trocar to be fixed in a locked position.

13. The trocar fixation assembly (100) according to any one of the preceding claims, wherein said second side (101b) of said dressing (101) comprises a backing layer (115) and wherein said dressing further comprises an absorbent pad (116) arranged between said backing layer (115) and said adhesive layer (103).

14. The trocar fixation assembly (100) according to any one of the preceding claims, wherein said aperture (104) is covered by an incision film (117) configured to be cut prior to or during insertion of a trocar (201) into said aperture (104).

15. The trocar fixation assembly (100) according to claim 14, wherein said incision film (117) comprises markings (118) indicating where and/or how to cut said incision film (117).

16. The trocar fixation assembly (100) according to any one of the preceding claims, wherein said dressing comprises a gripping tab (119) extending radially from an outer periphery of said dressing (101).

17. A trocar system (200) comprising the trocar fixation assembly (100) according to any one of the preceding claims and a trocar (201).
